# FASCICULE DE BREVET EUROPEEN

(11) **EP 0 871 419 B1**
(45) Date de publication et mention de la délivrance du brevet: **07.11.2001**
(21) Numéro de dépôt: 96942434.0
(22) Date de dépôt: 19.12.1996
(51) Int. Cl.: A61F 2/44, A61B 17/17, A61B 17/32, A61F 2/46

(54) **IMPLANT INTERVERTEBRAL DU TYPE CAGE INTERSOMATIQUE**
ZWISCHENWIRBELIMPLANTAT ALS WIRBELKÄFIG AUSGEBILDET
INTERBODY-TYPE INTERVERTEBRAL IMPLANT

(30) Priorité: 21.12.1995 FR 9515600
(43) Date de publication de la demande: 21.10.1998
(73) Titulaire: Medtronic Sofamor Danek, Inc., Memphis, TN 38132 (US)
(72) Inventeur: GROSSE, Arsène, F-67400 Illkirch-Graffenstaden (FR); BRAUN, Emmanuel, F-54000 Nancy (FR); DEHOUX, Emile, F-05100 Briançon (FR); DELEFORTRIE, Guido, B-5140 Ligny (BE); MUNTING, Everard, B-1390 Piez (BE)
(74) Mandataire: Neyret, Daniel
(86) Numéro de dépôt international: FR9602032
(87) Numéro de publication internationale: WO9723174

(56) Documents cités:
- EP-A- 0 421 485
- EP-A- 0 637 440
- WO-A-93/01771
- WO-A-94/17759
- WO-A-95/31947
- FR-A- 2 710 519
- US-A- 4 736 738
- US-A- 5 458 638

## Description

La présente invention concerne un implant intervertébral, du type couramment dénommé "cage intersomatique", permettant de réaliser l'immobilisation de deux vertèbres adjacentes. Elle concerne également trois instruments ancillaires pour la mise en place de cet implant. Les caractéristiques du préambule de la revendication 1 sont connues du document EP-A-0 421 485.

La dégénérescence d'un disque intervertébral conduit à une compression locale de la moelle épinière et des racines nerveuses. Il est alors nécessaire de restaurer l'espace intervertébral et d'immobiliser les vertèbres situées de part et d'autre du disque affecté.

Une technique consiste à implanter un greffon osseux entre les deux plateaux vertébraux, après ablation du disque. Un tel greffon résiste toutefois faiblement aux contraintes exercées par les mouvements du patient et risque de s'insérer à la longue dans l'une ou l'autre des vertèbres.

Il a alors été imaginé de placer le greffon dans un implant rigide, ou "cage intersomatique", ouvert à ses extrémités supérieure et inférieure. Un tel implant présente une hauteur supérieure à celle de l'espace intervertébral à restaurer, de sorte qu'il est nécessaire, lors de la préparation du site, de creuser l'os sous-chondral dur des plateaux vertébraux jusqu'à atteindre l'os spongieux. Après implantation, le greffon vient ainsi au contact de cet os spongieux et l'ostéo-intégration peut s'opérer.

Certaines cages ont une forme générale cylindrique ou tronconique. Ces cages ont pour inconvénient de présenter des risques d'insertion non négligeables dans l'une ou l'autre des vertèbres. En effet, elles reposent principalement sur de l'os spongieux et leur forme arrondie favorise cette insertion.

Il est certes possible de réduire les dimensions des ouvertures supérieures et inférieures de la cage, pour augmenter la surface d'appui contre l'os, mais cette réduction se fait au détriment de la surface de contact du greffon avec l'os, donc au détriment de la solidité de la fusion.

D'autres cages ont une forme parallélépipédique. Elles présentent des risques d'insertion moindres que lés cages cylindriques, grâce à leurs surfaces d'appui planes, et ont une hauteur inférieure à celle des cages cylindriques, ce qui permet une ablation moindre d'os sous-chondral.

Toutefois, l'aménagement d'un logement de section carrée ou rectangulaire implique le perçage d'un alésage puis une découpe dans l'os sous-chondral des quatre angles du logement au. moyen d'un ciseau emporte-pièce. La manipulation d'un tel instrument, à proximité de la moelle épinière et des racines nerveuses, est extrêmement délicate.

Il existe une cage de forme générale parallélélipédique, dont chacune des faces supérieure et inférieure comprend une nervure longitudinale faisant saillie de sa zone médiane. Des ouvertures sont aménagées au travers de ces nervures.

Cette cage est conformée. de telle sorte qu'après implantation, ses faces supérieure et inférieure portent contre la corticale des plateaux vertébraux et que lesdites nervures soient engagées dans l'os spongieux des vertèbres. Cet appui des faces supérieure et inférieure de la cage vise à limiter l'insertion de la cage dans l'os spongieux.

La cage comprend des entailles aménagées dans les angles formés par ses faces supérieure, inférieure et latérales. Ces entailles peuvent former conjointement des portions d'un filet hélicoïdal.

Un logement circulaire est percé entre les deux vertèbres à traiter, de manière à éliminer localement la corticale des plateaux vertébraux et à atteindre l'os spongieux. La cage est mise en place dans ce logement, par impaction ou par vissage.

Les faces supérieure et inférieure de la cage portent contre des zones d'os cortical amincies, puisque découpées en biseau lors du perçage du logement.

De plus, l'impaction de la cage conduit à léser ces zones d'appui cortical, et le vissage de la cage conduit à un alésage du logement aux dimensions desdites nervures, supérieures au diamètre du logement.

En outre, les surfaces d'appui des faces supérieure et inférieure de la cage sont réduites, notamment du fait de la présence des entailles précitées.

La présente invention vise à remédier à l'ensemble des inconvénients des cages intersomatiques de la technique antérieure.

Selon l'invention l'implant présente les caractéristiques de la revendication 1.

La forme curviligne des faces supérieures et inférieures permet l'aménagement du logement récepteur du corps de l'implant par perçage d'un ou plusieurs alésages au travers du disque affecté et des plateaux vertébraux, de manière à éliminer localement l'os sous-chondral de ces plateaux.

Une ou deux rainures sont aménagées sur un ou les deux côtés de ce logement pour recevoir la ou les protubérances de l'implant.

Le ou les perçages précités sont relativement faciles à réaliser, sans risques de lésions, et l'aménagement de la ou des rainures, uniquement à travers le disque, est également une opération relativement facile à réaliser.

La ou les protubérances viennent en appui contre des zones de l'os sous-chondral situées au-delà du logement, donc non lésées par l'aménagement de ce logement ou par l'introduction de l'implant dans celui-ci, et ont en outre des surfaces d'appui importantes.

Cette ou ces protubérances s'opposent ainsi parfaitement à toute insertion de l'implant dans l'os spongieux.

Dès lors, l'implant peut comprendre des ouvertures supérieure et inférieure de dimensions maximales, dont la surface est augmentée par la forme curviligne des faces supérieure et inférieure du corps de cet implant.

Ces ouvertures et cette forme curviligne permettent également une mise en compression du greffon et une possibilité de visualisation latérale de l'interface greffon-os spongieux par radiographie.

L'implant permet ainsi une parfaite fusion intervertébrale.

De préférence, l'implant comprend une protubérance placée, lorsqu'il est mis en place, sur le côté extérieur des vertèbres par rapport à l'axe du rachis, donc à distance de la moelle épinière et des centres nerveux. Tout risque de lésion est ainsi réduit au maximum.

Le corps de l'implant peut avoir une section transversale de forme circulaire. De préférence, toutefois, sa section transversale présente une forme annulaire ou ovale, avec la plus grande dimension de cet anneau ou de cet ovale placée verticalement lorsque l'implant est mis en place.

Cette forme annulaire ou ovale permet à l'implant d'avoir, pour une hauteur donnée, une largeur moindre qu'une cage de section circulaire. Le logement récepteur de cet implant est ainsi maintenu à distance de la moelle épinière et des centres nerveux situés le long de l'axe du rachis.

La protubérance peut avoir des surfaces d'appui parallèles, dans le cas d'un implant destiné à être placé entré des vertèbres à plateaux sensiblement parallèles. La protubérance peut également avoir des surfaces d'appui inclinées de manière à converger l'une vers l'autre en direction de l'extrémité postérieure de l'implant. Un tel implant est adapté à des vertèbres dont les plateaux ne sont pas parallèles, et permet de rétablir la lordose anatomique des vertèbres, qui existait avant la dégradation du disque.

Le corps de l'implant peut en outre présenter une section transversale constante, en particulier cylindrique, ou une section transversale se réduisant en direction de l'extrémité postérieure de l'implant. Le corps de l'implant participe également au rétablissement de la lordose anatomique des vertèbres.

En outre, le corps peut présenter une face postérieure inclinée de manière à ce que la surface inférieure du corps soit augmentée. Un tel implant peut être utilisé en cas de spondylolisthésis, et présente une surface d'appui maximale sur la vertèbre inférieure.

L'implant selon l'invention peut être mis en place par voie postérieure ou antérieure, et comprend des alésages taraudés aménagés dans ses faces postérieure et antérieure, pour son montage sur l'extrémité d'un instrument d'introduction et d'impaction. De préférence, ces alésages débouchent dans la cavité de l'implant recevant le greffon, pour permettre l'injection de copeaux d'os spongieux dans cette cavité, à l'aide de cet instrument, décrit plus loin.

L'invention concerne également trois instruments ancillaires utilisables pour la mise en place de l'implant.

Le premier de ces instruments est un guide de perçage comprenant une partie tubulaire apte à guider un foret de perçage et deux lames parallèles à extrémité libre tranchante, faisant saillie longitudinalement d'une extrémité de cette partie tubulaire, sur deux côtés opposés de celle-ci ; l'une de ces lames présente une hauteur et une épaisseur correspondant sensiblement à la hauteur maximale et à l'épaisseur de la protubérance de l'implant.

Ces lames sont destinées à être insérées dans le disque, avant perçage du logement devant recevoir le corps de l'implant. Elles permettent une parfaite immobilisation du guide de perçage par rapport au disque et aux vertèbres. La lame dont la hauteur et l'épaisseur correspondent sensiblement à la hauteur maximale et à l'épaisseur de la protubérance latérale de l'implant permet d'aménager, par cette simple insertion, la rainure destinée à recevoir cette protubérance. La deuxième lame permet, quant à elle, d'assurer la stabilité en rotation du guide de perçage.

Le second de ces instruments ancillaires est un ostéotome permettant le prélèvement du greffon osseux destiné à remplir l'implant. Cet ostéotome comprend une cavité de réception du greffon prélevé, dont l'entrée est délimitée par un bord libre tranchant, cette cavité ayant une forme correspondant sensiblement à la forme de la cavité de l'implant. Le greffon prélevé s'adapte ainsi précisément à la cavité de l'implant.

Le troisième instrument ancillaire est l'instrument précité d'introduction et d'impaction de l'implant entre les vertèbres. Selon l'invention, cet instrument est tubulaire et comprend un piston pouvant coulisser dans son alésage intérieur. Il permet ainsi d'opérer une injection de copeaux d'os spongieux à l'intérieur de la cavité de l'implant, de manière à assurer le parfait remplissage de cette cavité.

Pour sa bonne compréhension, l'invention est à nouveau décrite ci-dessous en référence au dessin schématique annexé représentant, à titre d'exemples non limitatifs, plusieurs variantes de réalisation de l'implant intervertébral qu'elle concerne, ainsi qu'une forme de réalisation des différents instruments ancillaires pour la mise en place de cet implant.
La figure 1 est une vue en perspective de l'implant, selon une première forme de réalisation ;
la figure 2 en est une vue en coupe selon la ligne II-II de la figure 1 ;
la figure 3 en est une vue de profil, après implantation entre deux vertèbres ;
la figure 4 est une vue postérieure de deux vertèbres après mise en place d'un implant sur le côté droit et perçage, sur le côté gauche, du logement destiné à recevoir un implant ;
la figure 5 est une vue de dessus de ces deux implants, après mise en place ;
la figure 6 est une vue en perspective d'un premier instrument ancillaire permettant la mise en place de cet implant ;
la figure 7 est une vue en bout de cet instrument, en cours d'utilisation ;
la figure 8 est une vue en bout de cet instrument, selon une variante ;
la figure 9 est une vue en perspective d'un deuxième instrument, permettant le prélèvement d'un greffon osseux ;
la figure 10 en est une vue en coupe selon la ligne X-X de la figure 9 ;
la figure 11 est une vue en coupe longitudinale d'un troisième instrument ancillaire permettant la mise en place de cet implant, à l'état démonté ;
la figure 12 est une vue partielle de cet instrument lors de la mise en place d'un implant, dans une première position ;
la figure 13 est une vue similaire à la figure 12, dans une deuxième position de l'instrument ;
les figures 14 à 19 sont des vues, similaires à la figure 1, de l'implant selon plusieurs variantes de réalisation, et
la figure 20 est une vue de profil de l'implant représenté à la figure 19, après implantation entre deux vertèbres, dans le cas d'une spondylolisthésis.

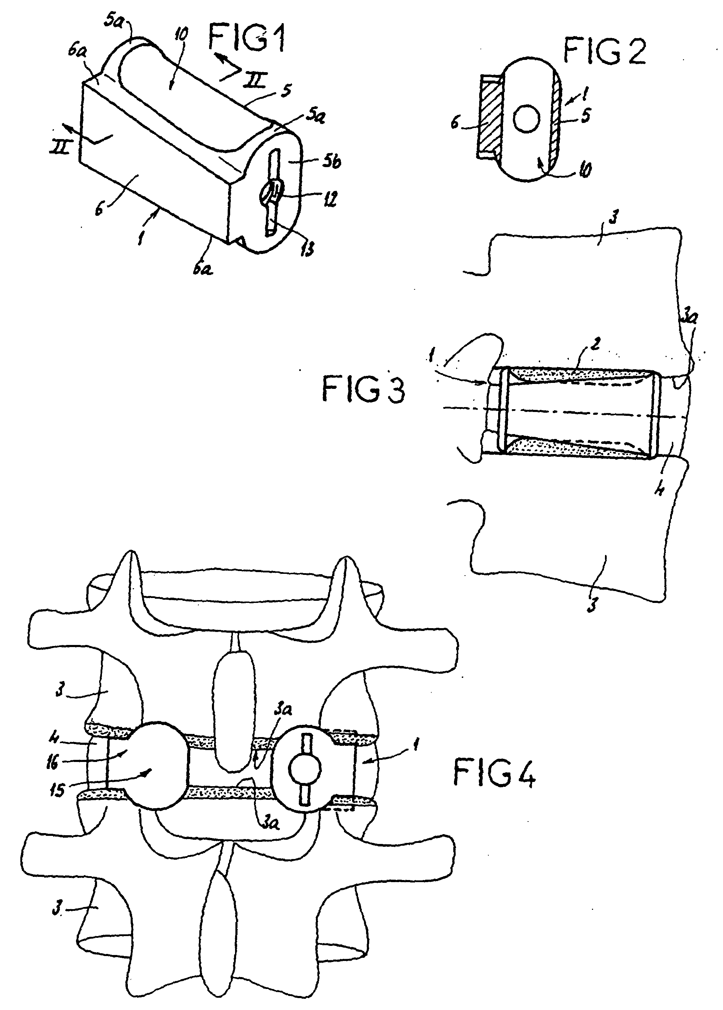

Les figures 1 et 2 représentent, sous différents angles, un implant intervertébral 1, du type couramment dénommé "cage intersomatique".

Ainsi que cela apparaît sur les figures 3 à 5, deux implants 1 sont destinés à recevoir chacun un greffon osseux 2 et à être insérés entre deux vertèbres 3 dont le disque 4 est détérioré. Les implants 1 permettent de restaurer l'espace intervertébral et de réaliser l'immobilisation relative des vertèbres 3.

L'implant 1 comprend un corps 5 et une protubérance latérale 6.

Le corps 5 présente une section transversale de forme annulaire et délimite intérieurement une cavité 10 de réception du greffon 2. Cette cavité 10 débouche sur l'extérieur par deux larges ouvertures aménagées dans les faces longitudinales arrondies 5a de ce corps 5.

Les deux faces d'extrémité 5b du corps 5 comprennent chacune un alésage taraudé central 12, et l'une d'elles comprend une rainure transversale 13.

La protubérance 6 forme une seule pièce avec le corps 5. Elle présente deux faces longitudinales 6a, qui convergent l'une vers l'autre en direction de l'une des faces 5b du corps 5, symétriquement par rapport à un plan longitudinal médian de la protubérance 6.

Il apparaît sur les figures 3 et 4 que le corps 5 a une hauteur supérieure à l'espace intervertébral à restaurer. L'aménagement du logement 15 destiné à recevoir ce corps 5 implique par conséquent de creuser non seulement le disque 4 mais également l'os sous-chondral des plateaux vertébraux 3a, jusqu'à atteindre l'os spongieux. Le greffon 2 peut ainsi venir au contact de cet os spongieux afin que l'ostéo-intégration puisse s'opérer.

Par contre, la hauteur de la protubérance 6 correspond sensiblement à la hauteur de l'espace intervertébral à restaurer, et cette protubérance est destinée à être engagée dans une rainure 16 aménagée sur le côté extérieur du logement 15, uniquement dans le disque 4. La protubérance 6 est ainsi insérée entre les plateaux vertébraux 3a, au niveau de surfaces non réséquées de ceux-ci, constituées d'os sous-chondral dur, ces plateaux 3a venant en appui contre les faces 6a.

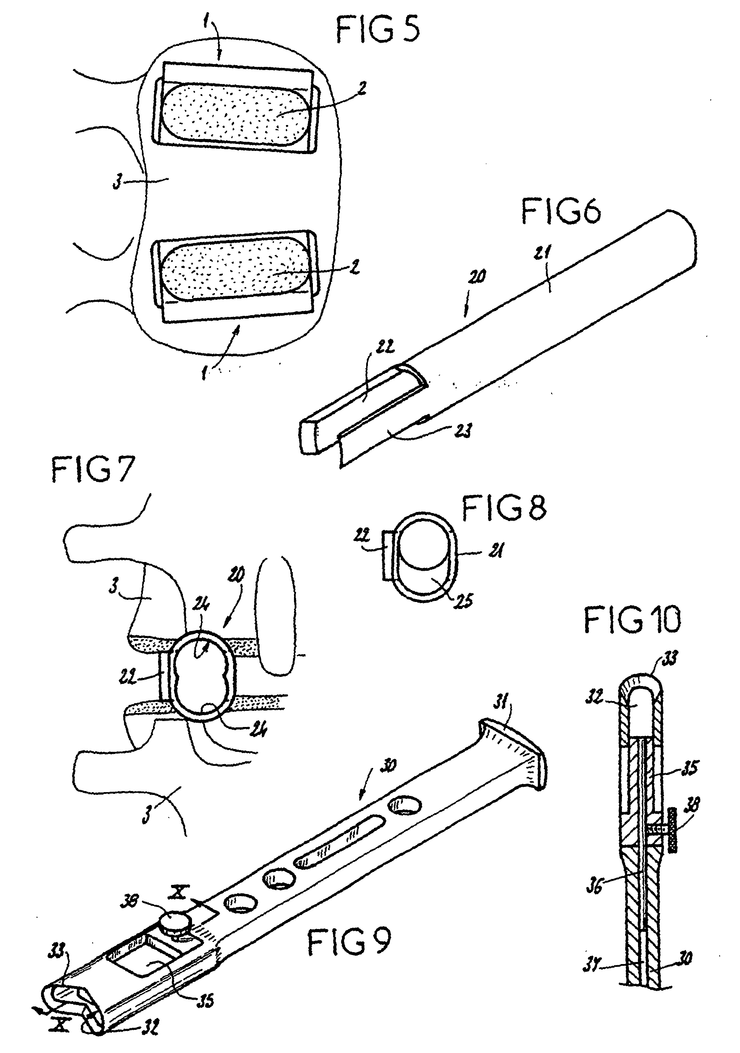

La figure 6 représente un guide de perçage 20 permettant d'aménager le logement 15 et la rainure 16. Ce guide de perçage 20 comprend une partie tubulaire 21 apte à guider un foret de perçage et deux lames parallèles 22,23 à extrémité libre tranchante. Ces lames 22,23 font saillie longitudinalement de l'une des extrémités de cette partie tubulaire 21, sur deux côtés opposés de celle-ci. La lame 22 présente une hauteur et une épaisseur correspondant sensiblement à la hauteur maximale et à l'épaisseur de la protubérance 6.

La partie tubulaire 21 est de forme annulaire et permet l'aménagement du logement 15, par le perçage de deux alésages parallèles décalés. Comme le montre la figure 7, cette partie tubulaire 21 comprend deux canons 24 partiellement sécants, de guidage du foret. La figure 8 montre que, selon une variante, cette partie 21 peut comprendre une cavité annulaire et une cale longitudinale amovible 25 en forme de croissant, permettant de délimiter successivement chacun des deux canons de perçage.

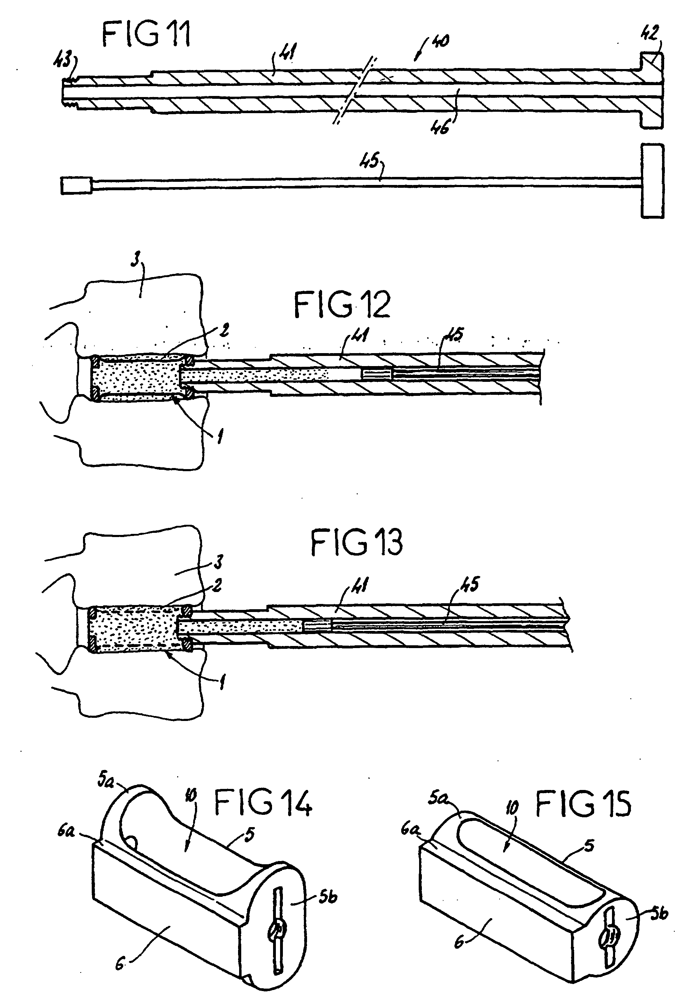

Les figures 9 et 10 représentent un ostéotome 30 permettant le prélèvement du greffon osseux 2. Cet ostéotome 30 comprend, à une extrémité, une tête de frappe 31 et, à son autre extrémité, une cavité 32 de réception du greffon prélevé, dont l'entrée est délimitée par un bord libre tranchant 33. Cette cavité 32 a une forme correspondant sensiblement à la forme de la cavité 10, de sorte que le greffon 2 prélevé s'adapte précisément à cette cavité.

L'ostéotome 30 comprend également un curseur 35 placé au fond de la cavité 32, guidé par une tige 36 coulissant dans un alésage 37, et pouvant être actionné grâce à un téton 38. Ce curseur 35 permet l'éjection facile du greffon 2 hors de la cavité 32.

Les figures 11 à 13 montrent un instrument 40 permettant l'introduction et l'impaction de l'implant 1 dans le logement 15 et la rainure 16.

Cet instrument 40 comprend un corps tubulaire 41, une tête de frappe 42 à une extrémité et un embout fileté 43 à son autre extrémité. Cet embout 43 peut être vissé dans l'un ou l'autre des alésages 12 de l'implant 1, selon que cette dernière est implantée par voie antérieure ou postérieure.

En outre, l'instrument 40 comprend une tige 45 formant un piston pouvant coulisser dans l'alésage intérieur 46 du corps 41.

En pratique, les vertèbres 3 sont distractées en vue du perçage des logements 15,16 puis de l'insertion des cages 1.

Comme le montre la figure 7, le guide de perçage 20 est tout d'abord impacté jusqu'à insertion complète des lames 22,23 dans le disque 4. Ces lames 22,23, une fois insérées, immobilisent parfaitement le guide de perçage 20. La lame 22 permet d'aménager la rainure 16 par cette simple insertion, tandis que la lame 23 assure la stabilité en rotation du guide de perçage 20.

L'ostéotome 30 permet le prélèvement du greffon 2, notamment dans la crête iliaque du patient. Le bord tranchant 33 assure la découpe de ce greffon 2 à la forme de la cavité 10 lorsqu'un martelage est exercé sur la tête de frappe 31. Le greffon obtenu est ainsi en un seul bloc.

Son transfert dans la cavité 10 est réalisé facilement et rapidement grâce au curseur 35.

Après perçage des deux alésages permettant d'aménager le logement 15 et retrait du guide 20, l'implant 1 est monté sur l'embout 43 de l'instrument 40 puis est impacté dans le logement 15 et la rainure 16. Une fois mis en place, une injection de copeaux d'os spongieux à l'intérieur de la cavité 10 peut être opérée par pression sur le piston 45, comme montré à 1a. figure 13, de manière à assurer le parfait remplissage de cette cavité 10.

La rainure 13 permet, dans le cas d'un positionnement défectueux de l'implant 1 lors de l'impaction, de faire pivoter l'implant 1 sur lui-même pour l'amener dans une position adéquate.

Les figures 14 à 19 montrent que de nombreuses variantes sont possibles dans la forme de l'implant 1. D'une manière générale, le corps 5 peut avoir une section transversale de forme circulaire (figures 15,18,19), annulaire (figures 14,16,17) ou ovale, avec la plus grande dimension de cet anneau ou de cet ovale placée verticalement lorsque l'implant 1 est mis en place.

Le corps 5 peut également présenter une section transversale constante (figures 14,15,19), ou une section transversale qui se réduit en direction de l'extrémité postérieure de l'implant 1 (figures 16,17,18), afin que ce corps 5 participe, conjointement à la protubérance 6, au rétablissement de la lordose anatomique des vertèbres 3.

La protubérance 6, quant à elle, peut avoir des surfaces d'appui 6a qui convergent en direction de l'extrémité postérieure de l'implant 1 (figures 17, 18,19), selon des angles pouvant varier de 1 à 15 degrés, ou peut avoir des surfaces d'appui 6a parallèles (figures 14,15,16) permettant la mise en place de l'implant entre des vertèbres à plateaux sensiblement parallèles.

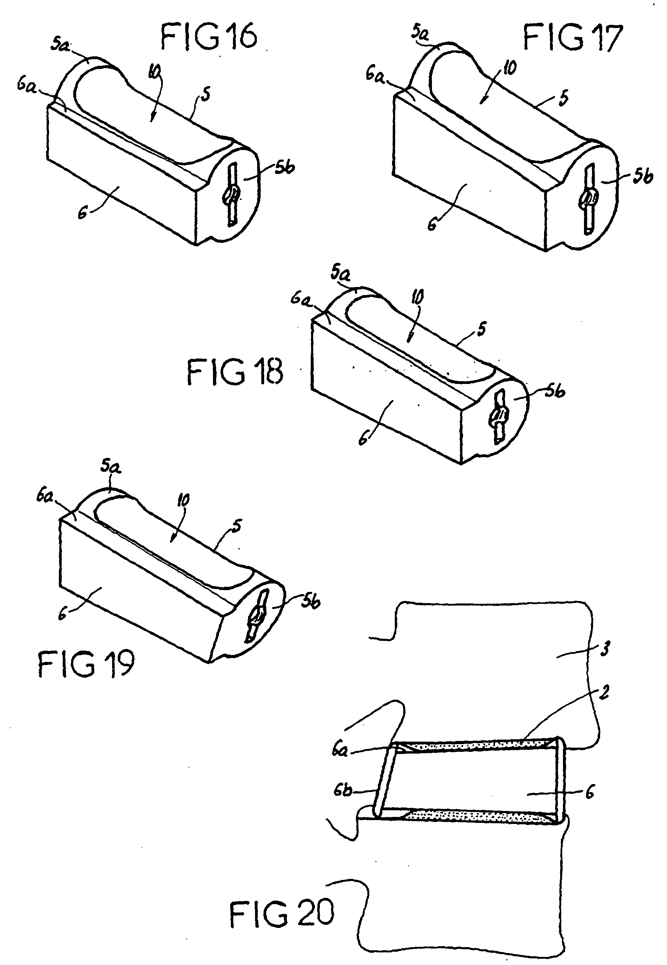

En outre, comme le montre la figure 19, le corps 5 peut présenter une face postérieure 5b inclinée de manière à augmenter la surface inférieure du corps 5. Un tel implant peut être mis en place en cas de spondylolisthésis, comme cela apparaît à la figure 20, et présente une surface d'appui maximale sur la vertèbre 3 inférieure.

Il va de soi que les ouvertures de l'implant peuvent occuper une très large partie des faces 5a, comme représenté, ou n'occuper qu'une partie plus réduite de celles-ci ; le corps 5 peut comporter une ou plusieurs cloisons transversales dans la cavité 10.

## Revendications

1. Implant intervertébral permettant de réaliser l'immobilisation de deux vertèbres adjacentes, comprenant un corps (5), dont les faces supérieure et inférieure (5a) sont destinées à venir au contact de l'os spongieux des plateaux vertébraux (3a) lorsque l'implant est mis en place, et au moins une protubérance latérale (6), dont la hauteur correspond sensiblement à celle de l'espace intervertébral à restaurer, cette protubérance (6) étant destinée à être insérée entre les plateaux vertébraux (3a), au niveau de surfaces constituées d'os sous-chondral dur,
les faces supérieures et inférieures (5a) de son corps (5) présentant un contour curviligne en section transversale, et
la protubérance (6) étant conformée de manière à faire saillie au-delà de l'enveloppe de ce corps (5), implant (1) **caractérisé en ce que** les faces supérieure et inférieure (5a) présentent une cavité (10) qui passe de la face supérieure à la face inférieure à travers du corps (5) et qui est destinée à recevoir un greffon osseux.

2. Implant selon la revendication 1, **caractérisé en ce qu'**il comprend une protubérance (6) placée, lorsqu'il est mis en place, sur le côté extérieur des vertèbres (3) par rapport à l'axe du rachis.

3. Implant selon la revendication 1 ou la revendication 2, **caractérisé en ce que** son corps (5) présente une section transversale de forme circulaire, annulaire ou ovale, avec la plus grande dimension de cet anneau ou de cet ovale placée verticalement lorsque l'implant (1) est mis en place.

4. Implant selon l'une des revendications 1 à 3, **caractérisé en ce que** sa protubérance (6) présente des surfaces d'appui (6a) parallèles, ou inclinées de manière à converger l'une vers l'autre en direction de l'extrémité postérieure de l'implant (1).

5. Implant selon l'une des revendications 1 à 4, **caractérisé en ce que** son corps (5) présente une section transversale constante, en particulier cylindrique, ou une section transversale se réduisant en direction de son extrémité postérieure.

6. Implant selon l'une des revendications 1 à 5, **caractérisé en ce que** son corps (5) présente une face postérieure (5b) inclinée de manière à ce que la surface inférieure du corps (5) soit augmentée.

7. Implant selon l'une des revendications 1 à 6, **caractérisé en ce qu'**il comprend des alésages (12) aménagés dans ses faces postérieure et antérieure (5b), ces alésages (12) débouchant dans la cavité (10) recevant le greffon (2).

8. Ensemble comprenant un implant intervertébral selon la revendication 1 et un guide de perçage pour l'aménagement du logement récepteur (15,16) de l'implant selon l'une des revendications 1 à 7, **caractérisé en ce qu'**il comprend une partie tubulaire (21) apte à guider un foret de perçage et deux lames parallèles (22,23) à extrémité libre tranchante, faisant saillie longitudinalement d'une extrémité de cette partie tubulaire (21), sur deux côtés opposés de celle-ci, l'une (22) de ces lames présentant une hauteur et une épaisseur correspondant sensiblement à la hauteur maximale et à l'épaisseur de la protubérance (6) de l'implant (1).

9. Ensemble comprenant un implant intervertébral selon la revendication 1 et un ostéotome permettant le prélèvement du greffon (2) destiné à remplir l'implant (1) selon l'une des revendications 1 à 7, **caractérisé en ce qu'**il comprend une cavité (32) de réception du greffon prélevé, dont l'entrée est délimitée par un bord libre tranchant (33), cette cavité (32) ayant une forme correspondant sensiblement à la forme de la cavité (10) de l'implant (1).

10. Ensemble comprenant un implant intervertébral selon la revendication 1 et un instrument permettant l'introduction entre les vertèbres et l'impaction de l'implant (1) selon la revendication 7, **caractérisé en ce qu'**il est tubulaire et **en ce qu'**il comprend une tige (45) formant un piston, pouvant coulisser dans l'alésage intérieur (46) de cet instrument (40), pour permettre d'opérer une injection de copeaux d'os spongieux à l'intérieur de la cavité (10) de l'implant (1).

## Patentansprüche

1. Zwischenwirbelimplantat zur Immobilisierung zweier benachbarter Wirbel, mit einem Körper (5), dessen oberen und unteren (5a) Flächen zur Bildung eines Kontraktes mit dem schwammigen Knochen der Wirbelscheiben (3a) beim Anordnen des Implantats an Ort und Stelle vorgesehen sind, und mit mindestens einem lateralen Vorsprung (6), dessen Höhe genau der Höhe eines wiederherzustellenden Zwischenwirbelraums entspricht, wobei dieser Vorsprung (6) zum Einfügen zwischen den Wirbelscheiben (3a) auf der Ebene der Oberflächen vorgesehen ist, die den harten Unterknorpelknochen bilden, wobei die oberen und unteren (5a) Flächen des Körpers (5) einen krummlinigen Umriss als Querschnitt aufweisen, und wobei der Vorsprung (6) in einer über die Hülle des Körpers (5) hinaus vorspringenden Art und Weise ausgebildet ist,
**dadurch gekennzeichnet, dass**
die obere und untere (5a) Fläche einen Hohlraum (10) aufweisen, der von der oberen Fläche zu der unteren Fläche durch den Körper (5) hindurch läuft und der zur Aufnahme eines Knochentransplantats vorgesehen ist.

2. Implantat nach Anspruch 1, **dadurch gekennzeichnet, dass** dasselbe einen Vorsprung (6) aufweist, der, wenn derselbe an Ort und Stelle plaziert wird, auf der äußeren Seite der Wirbel (3) bezüglich einer Wirbelsäulenachse plaziert ist.

3. Implantat nach Anspruch 1 oder 2, **dadurch gekennzeichnet, dass** der Körper (5) desselben einen kreisförmigen, ringförmigen oder ovalen Querschnitt aufweist, wobei die größte Abmessung dieses Rings oder dieses Ovals vertikal plaziert ist, wenn das Implantat (I) an Ort und Stelle plaziert ist.

4. Implantat nach einem der Ansprüche 1 bis 3, **dadurch gekennzeichnet, dass** der Vorsprung (6) parallele oder auf einer Art und Weise geneigte Stützoberflächen (6a) aufweist, dass dieselben aufeinander in einer Richtung eines hinteren Endes des Implantats (1) aufeinander zulaufen.

5. Implantat nach einem der Ansprüche 1 bis 4, **dadurch gekennzeichnet, dass** der Körper (5) desselben einen konstanten Querschnitt und insbesondere zylindrischen oder einen Querschnitt aufweist, der sich in der Richtung des hinteren Endes desselben reduziert.

6. Implantat nach einem der Ansprüche 1 bis 5, **dadurch gekennzeichnet, dass** der Körper (5) desselben eine hintere Oberfläche (5b) aufweist, die auf einer An und Weise geneigt ist, dass die untere Oberfläche des Körpers (5) vergrößert ist.

7. Implantat nach einem der Ansprüche 1 bis 6, **dadurch gekennzeichnet, dass** dasselbe Bohrungen (12) aufweist, die auf der hinteren und vorderen Fläche (5b) angeordnet sind, wobei die Bohrungen (12) in den Hohlraum (10), der das Transplantat (2) aufnimmt, offen sind.

8. Anordnung mit einem Zwischenwirbelimplantat nach Anspruch 1 und einer Bohrungsführung für die Anordnung eines Lagerungsbehäters (15, 16) des Implantats nach einem der Ansprüche 1 bis 7, **dadurch gekennzeichnet, dass** dieselbe ein röhrenförmiges Teil (21) zum Führen eines Bohrers und zwei parallele Streifen (22, 23) an freienschneidenden Enden aufweist, die einen länglichen Vorsprung von einem Ende dieses röhrenförmigeu Teils (21) auf zwei gegenüberliegenden Seiten desselben bilden, wobei einer (22) der Streifen eine Höhe und eine Dicke aufweist, die genau einer maximalen Höhe und einer Dicke des Vorsprungs (6) des Implantats (1) entsprechen.

9. Anordnung mit einem Zwischenwirbelimplantat nach Anspruch 1 und ein Knochenmßel, für die Entnahme von Transplantat (2) zu Füllen des Implantats (1) nach einem der Ansprüche 1 bis 7, **dadurch gekennzeichnet, dass** dieselbe einen Hohlraum (32) zur Aufnahme eines entnommenen Transplantats aufweist, dessen Eingang durch einen freien schneidenden Rand (33) begrenzt ist, wobei dieser Hohlraum (32) eine Form aufweist, die genau einer Form des Hohlraums (10) des Implantats (1) entspricht.

10. Anordnung mit einem Zwischenwirbelimplantat nach Anspruch 1 und einer Vorrichtung für die Einführung zwischen den Wirbeln und für die Implantation des Implantats (1) nach Anspruch 7, **dadurch gekennzeichnet, dass** dieselbe röhrenförmig ist und einen Stiel (45) aufweist, der einen Kolben bildet, der in der inneren Bohrung (46) dieser Vorrichtung (40) für eine Injektion von Spänen des schwammigen Knochen in das Innere des Hohlraums (10) des Implantats (1) verschiebbar ist.

## Claims

1. Intervertebral implant for immobilising two adjacent vertebrae, comprising a body (5) the top and bottom surfaces (5a) of which are intended to come into contact with the spongy bone of the vertebral plates (3a) when the implant is put into position, and at least one lateral protuberance (6) the height of which corresponds substantially to that of the intervertebral space which is to be restored, this protuberance (6) being intended to be inserted between the vertebral plates (3a), level with surfaces formed from hard subchondral bone, the top and bottom surfaces (5a) of the body (5) thereof having a curved contour in cross-section, and the protuberance (6) being shaped so as to project beyond the envelope of this body (5), said implant (1) being **characterised in that** the top and bottom surfaces (5a) have a cavity (10) which passes from the top surface to the bottom surface through the body (5) and is adapted to receive a bone graft.

2. Implant according to claim 1, **characterised in that** it comprises a protuberance (6) which, when in position, is situated on the outer side of the vertebrae (3) relative to the axis of the spine.

3. Implant according to claim 1 or 2, **characterised in that** its body (5) is circular, annular or oval in cross-section, with the largest dimension of this ring or oval being placed vertically when the implant (1) is in position.

4. Implant according to one of claims 1 to 3, **characterised in that** its protuberance (6) has bearing surfaces (6a) which are parallel or inclined to converge with each other towards the rear end of the implant (1).

5. Implant according to one of claims 1 to 4, **characterised in that** its body (5) has a constant, particularly cylindrical, cross-section or a cross-section which decreases towards its rear end.

6. Implant according to one of claims 1 to 5, **characterised in that** its body (5) has a rear surface (5b) inclined so that the bottom surface of the body (5) is enlarged.

7. Implant according to one of claims 1 to 6, **characterised in that** it comprises bores (12) provided in its front and rear surfaces (5b), these bores (12) opening into the cavity (10) which receives the graft (2).

8. Assembly comprising an intervertebral implant according to claim 1 and a drilling guide for forming the recess (15, 16) receiving the implant according to one of claims 1 to 7, **characterised in that** it comprises a tubular portion (21) adapted to guide a drill bit and two parallel plates (22, 23) with a free cutting end, projecting longitudinally from one end of said tubular portion (21), on two opposite sides thereof, one (22) of these plates having a height and thickness which substantially correspond to the maximum height and thickness of the protuberance (6) on the implant (1).

9. Assembly comprising an intervertebral implant according to claim 1 and an osteotome for taking the graft (2) intended to fill the implant (1) according to one of claims 1 to 7, **characterised in that** it comprises a cavity (32) for receiving the graft taken, the entrance to which is defined by a free cutting edge (33), this cavity (32) having a shape which substantially corresponds to the shape of the cavity (10) of the implant (1).

10. Assembly comprising an intervertebral implant according to claim 1 and an instrument for introducing the implant (1) according to claim 7 between the vertebrae and impacting it, **characterised in that** it is tubular and comprises a rod (45) forming a piston which is able to slide in the inner bore (46) of this instrument (40) to allow fragments of spongy bone to be injected into the interior of the cavity (10) of the implant (1).
